# EUROPEAN PATENT SPECIFICATION

(11) **EP 1 758 999 B1**
(45) Date of publication and mention of the grant of the patent: **09.03.2011**
(21) Application number: 05705076.7
(22) Date of filing: 06.01.2005
(51) Int. Cl.: C12N 15/11, C12N 15/86, A61K 48/00

(54) **METHODS OF INHIBITING TUMOR CELL PROLIFERATION WITH FOXM1 siRNA**
VERFAHREN ZUR INHIBIERUNG VON TUMORZELLWACHSTUM MIT FOXM1 SIRNS
PROCEDES POUR INHIBER LA PROLIFERATION DES CELLULES TUMORALES AVEC FOXM1 ARNSI

(30) Priority: 22.06.2004 US 582141 P
(43) Date of publication of application: 07.03.2007
(60) Divisional application: 10075609.7
(73) Proprietor: THE BOARD OF TRUSTEES OF THE UNIVERSITY OF ILLINOIS, Urbana, IL 61801 (US)
(72) Inventor: COSTA, Robert, Oak Park, IL 60304 (US); WANG, I-Ching, Cincinnati, OH 45220 (US)
(74) Representative: Grünecker, Kinkeldey, Stockmair & Schwanhäusser Anwaltssozietät
(86) International application number: PCT/US2005/000276
(87) International publication number: WO 2006/009575

(56) References cited:
- WO-A-2004/018676
- WO-A-2004/019761
- WO-A-2004/100977
- KALINICHENKO VLADIMIR V ET AL: "Foxm1b transcription factor is essential for development of hepatocellular carcinomas and is negatively regulated by the p19ARF tumor suppressor." GENES & DEVELOPMENT. 1 APR 2004, vol. 18, no. 7, 1 April 2004 (2004-04-01), pages 830-850, XP002338894 ISSN: 0890-9369
- POTENTE MICHAEL ET AL: "11,12-Epoxyeicosatrienoic acid-induced inhibition of FOXO factors promotes endothelial proliferation by down-regulating p27Kip1." THE JOURNAL OF BIOLOGICAL CHEMISTRY. 8 AUG 2003, vol. 278, no. 32, 8 August 2003 (2003-08-08), pages 29619-29625, XP002338895 ISSN: 0021-9258
- ECHEVERRI C. AND BROWN D.: "siRNA design: it's all in the algorithm" AMBION TECHNOTES, [Online] vol. 11, no. 3, May 2004 (2004-05), XP002338897 Retrieved from the Internet: URL:http://www.ambion.com/techlib/tn/113/1 4.html> [retrieved on 2005-08-02]
- BROWN D.: "RNAi as a tool for mammalian gene analysis: applications of siRNAs" AMBION TECHNOTES, [Online] vol. 10, no. 5, October 2003 (2003-10), XP002338898 Retrieved from the Internet: URL:http://www.ambion.com/techlib/tn/105/1 4.html> [retrieved on 2005-08-02]
- MILHAVET OLLIVIER ET AL: "RNA interference in biology and medicine." PHARMACOLOGICAL REVIEWS. DEC 2003, vol. 55, no. 4, December 2003 (2003-12), pages 629-648, XP002338943 ISSN: 0031-6997
- KATOH M ET AL: "HUMAN FOX GENE FAMILY (REVIEW)" INTERNATIONAL JOURNAL OF ONCOLOGY, EDITORIAL ACADEMY OF THE INTERNATIONAL JOURNAL OF ONCOLOGY,, GR, vol. 25, no. 5, November 2004 (2004-11), pages 1495-1500, XP009049979 ISSN: 1019-6439
- RYE P D ET AL: "Interfering with cancer: a brief outline of advances in RNA interference in oncology." TUMOUR BIOLOGY : THE JOURNAL OF THE INTERNATIONAL SOCIETY FOR ONCODEVELOPMENTAL BIOLOGY AND MEDICINE. 2004 SEP-DEC, vol. 25, no. 5-6, September 2004 (2004-09), pages 329-336, XP002338899 ISSN: 1010-4283
- ELBASHIR S M ET AL: "Duplexes of 21-nucleotide RNAs mediate RNA interference in cultured mammalian cells" NATURE, NATURE PUBLISHING GROUP, LONDON, UK, vol. 411, no. 6836, 24 May 2001 (2001-05-24), pages 494-498, XP002529540 ISSN: 0028-0836
- MIYAGISHI MAKOTO ET AL: "U6 promoter-driven siRNAs with four uridine 3' overhangs efficiently suppress targeted gene expression in mammalian cells." NATURE BIOTECHNOLOGY MAY 2002, vol. 20, no. 5, May 2002 (2002-05), pages 497-500, XP002965488 ISSN: 1087-0156

## Description

### BACKGROUND OF THE INVENTION

This application is related to and claims priority to U.S. provisional application Serial No. 60/582,141 filed June 22, 2004.

These studies were funded by grants from the National Institute on Aging, grant number AG21842-02, and the National Institute of Diabetes and Digestive and Kidney Diseases, grant number DK54687-06. The U.S. government may have certain rights to this invention.

### 1. Field of the Invention

The invention relates to siRNA molecules inhibiting tumor cell proliferation by inhibiting FoxMIB activity and uses thereof.

### 2. Background of the Related Art

The Forkhead box transcription factors have been implicated in regulating cellular longevity and proliferative capacity. Such studies include a finding of increased longevity in *C*. *elegans* bearing a mutant daf-2 gene, which encodes the worm homolog of the insulin/Insulin-like Growth Factor 1 (IGF1) receptor (Lin et al., 1997, Science 278: 1319-1322; Ogg et a/., 1997, Nature 389: 994-999). Disruption of the daf-2 gene abolishes insulin-mediated activation of the phosphatidylinositol 3-kinase (PI3K) - protein kinase B/Akt (Akt) signal transduction pathway and prevents inhibition of the forkhead transcription factor daf-16 (corresponding to mammalian homologs FoxO1 or Fkhr) (Paradis and Ruvkun, 1998, Genes Dev. 12: 2488-2498). Activation of the PI3K/Akt pathway phosphorylates the C-terminus of the Daf-16 (FoxO1; Fkhr) gene product and mediates its nuclear export into the cytoplasm, thus preventing FoxO1 transcriptional activation of target genes (Biggs et al., 1999, Proc. Natl. Acad. Sci. USA 96: 7421-7426; Brunet et al., 1999, Cell 96: 857-68; Guo et al., 1999, J. Biol. Chem. 274: 17184-17192).

More recent studies of *Daf-2⁻ C. elegans* mutants have demonstrated that Daf-16 stimulates expression of genes that limit oxidative stress (Barsyte et al., 2001, FASEB J. 15: 627-634; Honda et al., 1999, FASEB J. 13: 1385-1393; Wolkow et al., 2000, Science 290: 147-150) and that the mammalian *FoxO1* gene could functionally replace the *Daf-16* gene in *C*. *elegans* (Lee et al., 2001, Curr. Biol. 11: 1950-1957). In proliferating mammalian cells, the PI3K/Akt signal transduction pathway is essential for G1 to S-phase progression because it prevents transcriptional activity of the FoxO1 and FoxO3 proteins, which stimulate expression of the CDK inhibitor p27 ^{kip1} gene (Medema et al., 2000, Nature 404: 782-787). Moreover, genetic studies in budding yeast demonstrated that forkhead Fkh1 and Fkh2 proteins are components of a transcription factor complex that regulates expression of genes critical for progression into mitosis (Hollenhorst et al., 2001, Genes Dev. 15: 2445-2456; Koranda et al., 2000, Nature 406: 94-98; Kumar et al., 2000, Curr. Biol. 10: 896-906; Pic et al., 2000, EMBO J. 19: 3750-3761).

Forkhead Box M1B (FoxM1B or FoxM1) transcription factor (also known as *Trident* and *HFH-11B*) is a proliferation-specific transcription factor that shares 39% amino acid homology with the HNF-3 winged helix DNA binding domain. The molecule also contains a potent C-terminal transcriptional activation domain that possesses several phosphorylation sites for M-phase specific kinases as well as PEST sequences that mediate rapid protein degradation (Korver et al., 1997, Nucleic Acids Res. 25: 1715-1719; Korver et al., 1997, Genomics 46: 435-442; Yao et al., 1997, J. Biol. Chem. 272: 19827-19836; Ye et al., 1997, Mol. Cell Biol. 17: 1626-1641).

*In situ* hybridization studies have shown that FoxM1B is expressed in embryonic liver, intestine, lung, and renal pelvis (Ye et al., 1997, Mol. Cell Biol. 17: 1626-1641). In adult tissue, however, FoxM1B is not expressed in postmitotic, differentiated cells of the liver and lung, although it is expressed in proliferating cells of the thymus, testis, small intestine, and colon (*Id*)*.* FoxM1B expression is reactivated in the liver prior to hepatocyte DNA replication following regeneration induced by partial hepatectomy (*Id*)*.*

FoxM1B is expressed in several tumor-derived epithelial cell lines and its expression is induced by serum prior to the G₁/S transition (Korver et al., 1997, Nucleic Acids Res. 25: 1715-1719; Korver et al., 1997, Genomics 46: 435-442; Yao et al., 1997, J. Biol. Chem. 272: 19827-19836; Ye et al., 1997, Mol. Cell Biol. 17: 1626-1641). Consistent with the role of FoxM1b in cell cycle progression, elevated FoxM1B levels are found in numerous actively-proliferating tumor cell lines (Korver et al., 1997, Nucleic Acids Res. 25: 1715-1719; Yao et al., 1997, J. Biol. Chem. 272: 19827-36; Ye et al., 1997, Mol. Cell Biol. 17: 1626-1641). Increased nuclear staining of FoxM1B was also found in human basal cell carcinomas (Teh et al., 2002, Cancer Res. 62: 4773-80), suggesting that FoxM1B is required for cellular proliferation in human cancers.

These studies and others suggest that FoxM1b plays some role in human cancers. FoxM1B, therefore, would provide an attractive target for anti-cancer therapies because FoxM1B expression typically declines during normal aging (see co-owned and copending PCT Application No. PCT/US03/27098, filed August 28, 2003 ). Thus, FoxM1B might provide a selective target that is more active in tumor cells than in normal cells, particularly terminally-differentiated, aged or aging normal cells that surround a tumor, allowing tumor cells to be treated while minimizing the deleterious side-effects of such compounds on normal cells.

### SUMMARY OF THE INVENTION

The invention provides:
1. siRNA molecule that inhibits FoxM1 B activity in a tumor cell, wherein the siRNA comprises a nucleotide sequence shown in SEQ ID NO. 1, 2, 3 or 4.
2. siRNA molecule of 1, wherein the siRNA comprises a single self-complementary RNA strand.
3. siRNA molecule of 1, wherein the siRNA comprises two separate complementary RNA strands.
4. Expression vector comprising a nucleotide sequence encoding the siRNA molecule of 1.
5. Expression vector of 4, wherein the expression vector is a viral vector.
6. Viral vector of 5, wherein the viral vector is an adeno-associated virus, retrovirus, adenovirus, or alphavirus.
7. Use of the siRNA molecule of 1 for the preparation of a medicament for treating a cancer patient.
8. Use of the siRNA molecule of 1 for the preparation of a medicament for inhibiting proliferation of a tumor cell, wherein the cell is contacted with the siRNA.
9. Pharmaceutical composition comprising the siRNA molecule according to 1.
10. Use of the pharmaceutical composition of 9 for the preparation of a medicament for treating a cancer patient.
11. Use of the pharmaceutical composition of 9 for the preparation of a medicament for inhibiting proliferation of a tumor cell, wherein the cell is contacted with the siRNA.
12. siRNA molecule of 1 for use in the treatment of cancer.
13. siRNA molecule of 1 for use in inhibiting proliferation of a tumor cell.
14. Pharmaceutical composition of 9 for use in treating cancer.
15. Pharmaceutical composition of 9 for use in inhibiting proliferation of a tumor cell.
16. Method of inhibiting proliferation of a tumor cell comprising the step of contacting the cell with the siRNA molecule of 1, wherein the method is not a method of treatment of the human or animal body by surgery or therapy.

Specific preferred embodiments of the invention will become evident from the following more detailed description of certain preferred embodiments and the claims.

### DETAILED DESCRIPTION OF THE DRAWINGS

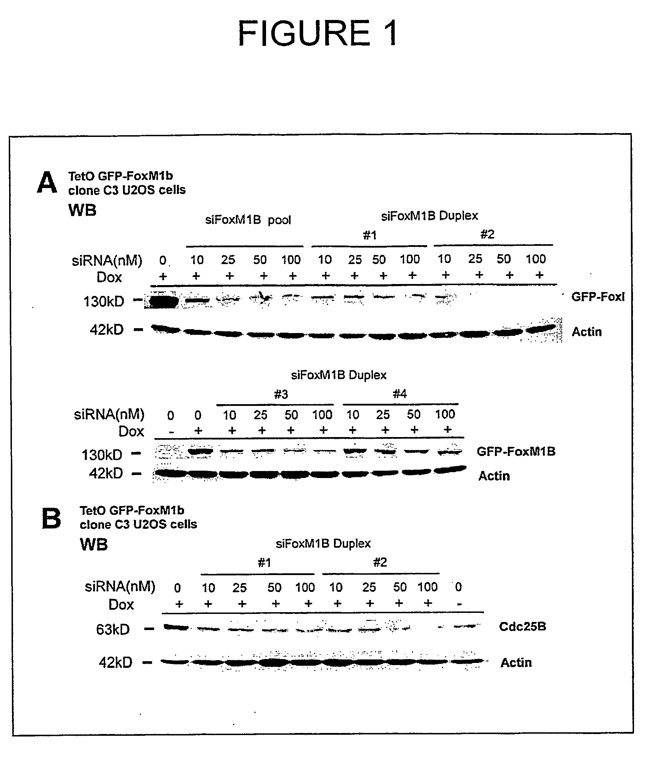

Figures 1A and 1B show that transfection of FoxM1b siRNAs inhibits expression of GFP-FoxM1b protein and its target Cdc25B protein as described in Example 1. Figure 1A: Dose response curve of FoxM1b siRNAs inhibition of GFP-FoxM1b protein levels. Increasing amounts of either the individual FoxM1b siRNAs (siFoxM1b #1 to #4) or the siFoxM1b pool were transfected into the TetO-GFP-FoxM1b clone C3 U2OS cell line following Doxycycline induction of the GFP-FoxM1b protein. Protein extracts were prepared 48 hours after transfection and then analyzed for GFP-FoxM1b protein levels by Western Blot analysis using a monoclonal antibody against GFP. Figure 1B: FoxM1b siRNA #2 effectively inhibits expression of the FoxM1b target Cdc25B protein. Transfection of increasing amounts of either siFoxM1b #1 or siFoxM1b #2 into Dox treated clone C3 U2OS cell line demonstrated that siFoxM1b #2 effectively diminished expression of the Cdc25B protein.

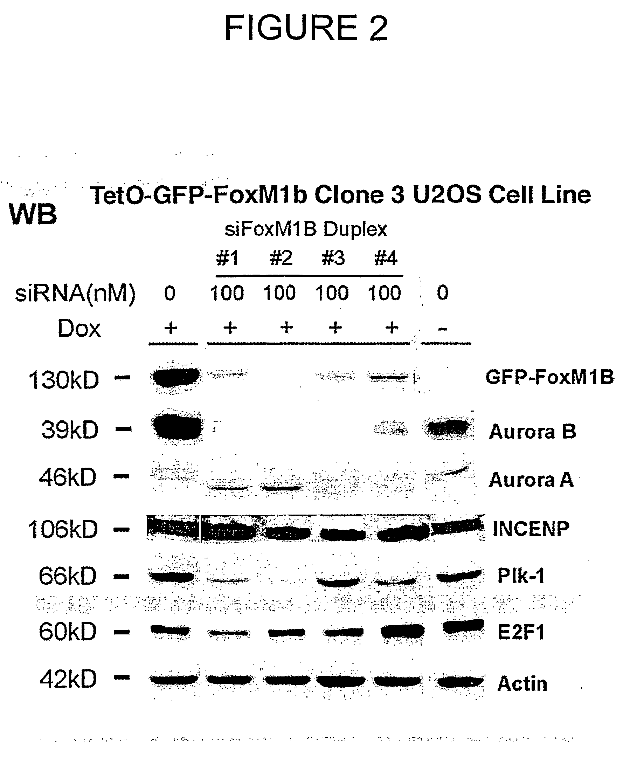

Figure 2 shows that transfection of FoxM1b siRNAs inhibits expression of GFP-FoxM1b protein and its potential targets involved in mitosis. The Clone C3 U2OS cell line were transfected with equal amounts (100 nM) of FoxM1b siRNAs #1, #2, #3 or #4, induced for GFP-FoxM1b with doxycycline (Dox) and protein extracts were isolated 48 hours after transfection. Western blot analysis was used to measure induced levels of GFP-FoxM1b protein and potential FoxM1b target proteins involved in orchestrating mitosis. Western blot analysis were performed with antibodies against the GFP protein, Aurora B kinase, Aurora A kinase, Inner Centromere Protein (INCENP), Polo-Like Kinase 1 (Plk-1) and the proliferation-specific E2F1 transcription factor.

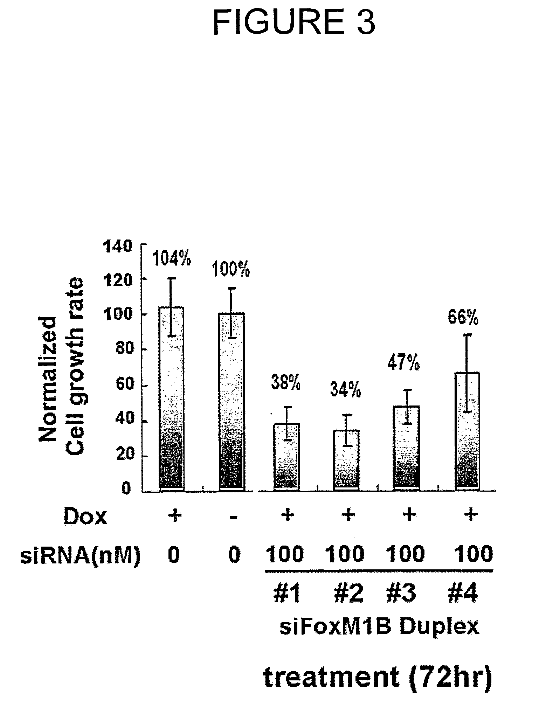

Figure 3 shows that transfection of FoxM1b siRNAs into U2OS cells decreases the rate of cell growth. U2OS cells were transfected with 100 nM of FoxM1b siRNAs #1, #2, #3 or #4 and cells were trypsinized 72 hours later and counted. Triplicate plates were used to determine the mean percentage of cells normalized to untreated ± standard deviation (SD).

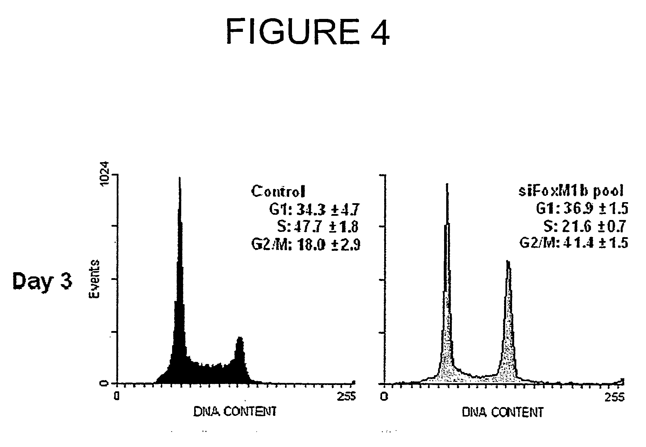

Figure 4 shows that transfection of FoxM1b siRNAs into U2OS cells caused accumulation of cells in the G2/M phase of the cell cycle as described in Example 1. U20S cells were transfected with 100 nM of FoxM1b siRNAs pool (mixture of siFoxM1b #1, #2, #3 and #4), cells were trypsinized 3 days later, fixed in ethanol, stained with propidium iodide and then analyzed for cell cycle progression by flow cytometry.

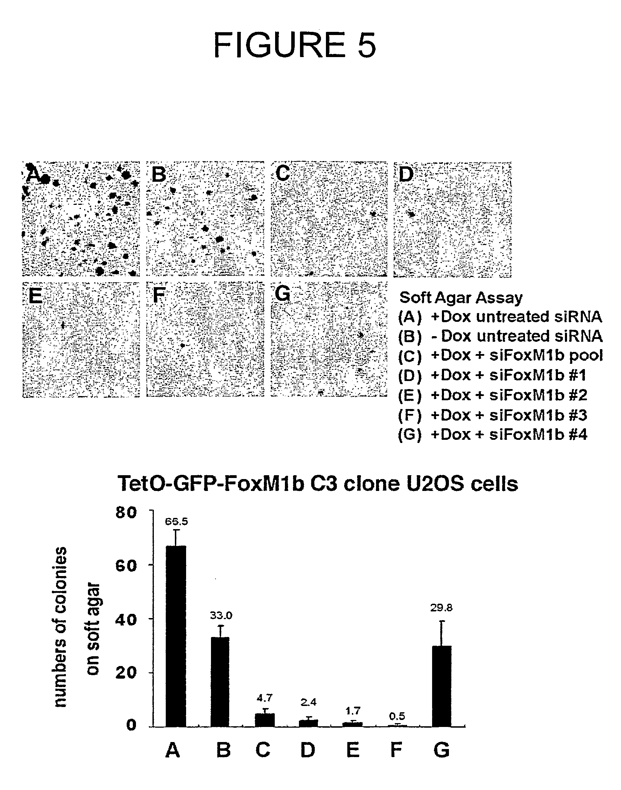

Figures 5A through 5G show that transfection of FoxM1b siRNAs into C3 clone U2OS cells diminishes anchorage-independent growth on soft agar. The Clone C3 U2OS cell line were transfected with 100 nM of FoxM1b siRNA pool (Figure 5C) or #1 (Figure 5D), #2 (Figure 5E), #3 (Figure 5F) or #4 (Figure 5G) and then induced for GFP-FoxM1b with doxycycline (Dox), with the results tabulated as shown in the bar graph in Figure 5. Control included Clone C3 U2OS cell line that was either induced (Figure 5A) or not induced (Figure 5B) with Dox. One day after FoxM1b siRNA transfection the cells assayed for anchorage-independent cell growth by plating them on soft agar for two weeks as described in Kalinichenko et al. (2004, Genes & Dev. 18: 830-850). Triplicate plates were used to count colonies and determine the mean number of colonies ± standard deviation (SD).

### DETAILED DESCRIPTION OF THE PREFERRED EMBODIMENTS

Conventional techniques well known to those with skill in the art were used for recombinant DNA production, oligonucleotide synthesis, and tissue culture and cell transformation (*e.g*., electroporation, lipofection) procedures. Enzymatic reactions and purification techniques were performed according to manufacturers' specifications or as commonly accomplished in the art or as described herein. The techniques and procedures were generally performed according to conventional methods well known in the art and as described in various general and more specific references that are cited and discussed throughout the present specification. *See e.g.,* Sambrook et al., 2001, MOLECULAR CLONING: A LABORATORY MANUAL, 3d ed., Cold Spring Harbor Laboratory Press, Cold Spring Harbor, N.Y. Unless specific definitions are provided, the nomenclature utilized in connection with, and the laboratory procedures and techniques of, molecular biology, genetic engineering, analytical chemistry, synthetic organic chemistry, and medicinal and pharmaceutical chemistry described herein are those well known and commonly used in the art. Standard techniques can be used for chemical syntheses, chemical analyses, and treatment of patients.

Unless otherwise required by context, singular terms shall include pluralities and plural terms shall include the singular.

### Definitions

As utilized in accordance with the present disclosure, the following terms, unless otherwise indicated, shall be understood to have the following meanings:

The invention describes isolated polynucleotides, particularly polynucleotides encoding or a portion of FoxM1B. As used herein, the term "isolated polynucleotide" means a polynucleotide of genomic, cDNA, or synthetic origin or a combination thereof, which by virtue of its source the "isolated polynucleotide" (1) is not associated with all or a portion of a polynucleotide in which the "isolated polynucleotide" is found in nature, (2) is linked to a polynucleotide which it is not linked to in nature, or (3) does not occur in nature as part of a larger sequence.

Unless specified otherwise, the left-hand end of single-stranded polynucleotide sequences is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

The term "polynucleotide" as used herein means a polymeric form of nucleotides that are at least 10 bases in length. In certain embodiments, the bases may be ribonucleotides or deoxyribonucleotides or a modified form of either type of nucleotide. The term includes single and double stranded forms of DNA.

The term "oligonucleotide" as used herein includes naturally occurring, and modified nucleotides linked together by naturally occurring, and/or non-naturally occurring oligonucleotide linkages. Oligonucleotides are a polynucleotide subset generally comprising no more than 200 nucleotides. In certain embodiments, oligonucleotides are 10 to 60 nucleotides in length. In certain embodiments, oligonucleotides are 12, 13, 14, 15, 16, 17, 18, 19, or 20 to 40 bases in length. Oligonucleotides can be single stranded, *e.g*. for use in the construction of a gene mutant using site directed mutagenesis techniques. Oligonucleotides may be sense or antisense oligonucleotides. An oligonucleotide can include a detectable label, such as a radiolabel, a fluorescent label, an antigenic label or a hapten.

The term "naturally occurring nucleotides" includes deoxyribonucleotides and ribonucleotides. The term "modified nucleotides" includes nucleotides with modified or substituted sugar groups and the like. The term "oligonucleotide linkages" includes oligonucleotides linkages such as phosphate, phosphorothioate, phosphorodithioate, phosphoroselenoate, phosphorodiselenoate, phosphoroanilothioate, phoshoraniladate, phosphoroamidate, and the like. *See, e.g.,* LaPlanche et al., 1986, Nucl. Acids Res. 14: 9081; Stec et al., 1984, J. Am. Chem. Soc. 106: 6077; Stein et a/., 1988, Nucl. Acids Res. 16: 3209; Zon et al., 1991, Anti-Cancer Drug Design 6: 539; Zon et al., 1991, OLIGONUCLEOTIDES AND ANALOGUES: A PRACTICAL APPROACH, (F. Eckstein, cd.), Oxford University Press, Oxford England, pp. 87-108; Stec et al., U.S. Pat. No. 5,151,510; Uhlmann and Peyman, 1990, Chemical Reviews 90: 543.

The term "vector" is used to refer to a molecule (*e.g*., nucleic acid, plasmid, or virus) used to transfer coding information to a host cell or a target cell. Viral vectors suitable for the methods of the invention include those derived from, for example, adenovirus, adeno-associated virus, retroviruses, herpes simplex virus, or vaccinia virus.

The term "expression vector" refers to a vector that is suitable for transformation of a host cell or a target cell and contains nucleic acid sequences comprising control sequences that direct and/or control the expression of inserted nucleic acid sequences. The term "expression" includes, but is not limited to, processes such as transcription and RNA splicing, if introns are present.

An expression vector of the invention can comprise a DNA or RNA sequence having a coding sequence that is operatively linked to a control sequence. The term "control sequence" or "control element" as used herein refers to polynucleotide sequences that can effect the expression and processing of coding sequences to which they are ligated. The nature of such control sequences may differ depending upon the host organism. According to certain embodiments, control sequences for prokaryotes may include promoters, repressors, operators, ribosomal binding sites, and transcription termination sequences and antisense mRNA. According to certain embodiments, control sequences for eukaryotes may include promoters, enhancers and transcription termination sequences, or sequences that regulate protein degradation, mRNA degradation, nuclear localization, nuclear export, cytoplasmic retention, protein phosphorylation, protein acetylation, protein sumolation, or RNA inhibition (RNAi). In certain embodiments, "control sequences" can include leader sequences and/or fusion partner sequences. "Control sequences" are "operatively linked" to a coding sequence when the "control sequence" effects expression and processing of coding sequences to which they are ligated.

As used herein, the phrase "tissue specific promoters" refers to nucleic acid sequences comprising control sequences that are capable of directing transcription of a coding sequence and that are activated specifically within a specific cell type. For example, liver specific promoters that drive expression of genes in liver cells include, but are not limited to, promoters from genes encoding human or mouse α1-antitrypsin, albumin promoter, serum amyloid A, transthyretin, hepatocyte nuclear factor 6, and major urinary protein (MUP).

Typically, expression vectors used in a host cells or target cell contain sequences for vector maintenance and for expression of exogenous nucleotide sequences. Such sequences, collectively referred to as "flanking sequences" in certain embodiments will typically include one or more of the following nucleotide sequences: a promoter, one or more enhancer sequences, a transcriptional termination sequence, a complete intron sequence containing a donor and acceptor splice site, a ribosome binding site, a polyadenylation signal sequence, a polylinker region comprising one or a plurality of restriction endonuclease sites for inserting nucleic acid encoding an siRNA to be expressed, and a selectable marker element.

Flanking sequences may be homologous (*i.e*., from the same species and/or strain as the host cell or the target cell), heterologous (*i.e.*, from a species other than the host cell or the target cell species or strain), hybrid (*i.e*., a combination of flanking sequences from more than one source), synthetic or native. As such, the source of a flanking sequence may be any prokaryotic or eukaryotic organism, any vertebrate or invertebrate organism, or any plant, provided that the flanking sequence is functional in, and can be activated by, the host cell or the target cell machinery.

Flanking sequences useful in the vectors of this invention may be obtained by any of several methods well known in the art. Typically, flanking sequences useful herein will have been previously identified by mapping and/or by restriction endonuclease digestion and can thus be isolated from the proper tissue source using the appropriate restriction endonucleases. In some cases, the full nucleotide sequence of a flanking sequence may be known. The flanking sequence also may be synthesized using the methods described herein for nucleic acid synthesis or cloning.

Where all or only a portion of the flanking sequence is known, it may be obtained using *in vitro* amplification methods such as polymerase chain reaction (PCR) and/or by screening a genomic library with a suitable oligonucleotide and/or flanking sequence fragment from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen^{®} column chromatography (Chatsworth, CA), or other methods known to the skilled artisan. The selection of suitable enzymes to accomplish this purpose is readily apparent to one of ordinary skill in the art.

A transcription termination sequence is typically located 3' to the end of a polypeptide-coding region and serves to terminate transcription. Usually, a transcription termination sequence in prokaryotic cells is a G-C rich fragment followed by a poly-T sequence. While the sequence is easily cloned from a library or even purchased commercially as part of a vector, it can also be readily synthesized using methods for nucleic acid synthesis such as those described herein. In eukaryotes, the sequence AAUAAA (SEQ ID NO: 5) functions both as a transcription termination signal and as a poly A signal required for endonuclease cleavage followed by the addition of poly A residues (usually consisting of about 200 A residues).

The expression and cloning vectors of the present invention will typically contain a promoter that is recognized by the host organism and operatively linked to nucleic acid encoding the FoxM1B protein. Promoters are untranscribed sequences located upstream (*i.e.,* 5') to the start codon of a structural gene (generally within about 100 to 1000 bp) that control transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no experimental control over gene expression. A large number of promoters, recognized by a variety of potential host cells or target cells, are well known.

Suitable promoters for use with mammalian cells are well known and include, but are not limited to, those obtained from the genomes of eukaryotic viruses such as polyoma virus, fowlpox virus, adenovirus (such as Adenovirus 2), bovine papilloma virus, avian sarcoma virus, cytomegalovirus, retroviruses, hepatitis-B virus and most preferably Simian Virus 40 (SV40). Other suitable mammalian promoters include heterologous mammalian promoters, for example, heat-shock promoters and the actin promoter.

Particular promoters useful in the practice of the recombinant expression vectors of the invention include, but are not limited to: the SV40 early promoter region (Bemoist and Chambon, 1981, Nature 290: 304-10); the CMV promoter; the promoter contained in the 3' long terminal repeat of Rous sarcoma virus (Yamamoto, et al., 1980, Cell 22: 787-97); the herpes thymidine kinase promoter (Wagner et al., 1981, Proc. Natl. Acad. Sci. U.S.A. 78: 1444-45); and the regulatory sequences of the metallothionine gene (Brinster et al., 1982, Nature 296: 39-42). Also of interest are the following animal transcriptional control regions, which exhibit tissue specificity and have been utilized in transgenic animals: the elastase I gene control region that is active in pancreatic acinar cells (Swift et al., 1984, Cell 38: 639-46; Ornitz et al., 1986, Cold Spring Harbor Symp. Quant. Biol. 50: 399-409; MacDonald, 1987, Hepatology 7: 425-515); the insulin gene control region that is active in pancreatic beta cells (Hanahan, 1985, Nature 315: 115-22); the mouse mammary tumor virus control region that is active in testicular, breast, lymphoid and mast cells (Leder et al., 1986, Cell 45: 485-95); the beta-globin gene control region that is active in myeloid cells (Mogram et al., 1985, Nature 315: 338-40; Kollias et al., 1986, Cell 46: 89-94); the myelin basic protein gene control region that is active in oligodendrocyte cells in the brain (Readhead et al., 1987, Cell 48: 703-12); the myosin light chain-2 gene control region that is active in skeletal muscle (Sani, 1985, Nature 314: 283-86); the gonadotropic releasing hormone gene control region that is active in the hypothalamus (Mason et al., 1986, Science 234: 1372-78); and most particularly the immunoglobulin gene control region that is active in lymphoid cells (Grosschedl et al., 1984, Cell 38: 647-58; Adames et al., 1985, Nature 318: 533-38; Alexander et al., 1987, Mol. Cell Biol. 7: 1436-44).

Preferably, the promoter of an expression vector of the invention is active in the tissue from which a target or host cell is derived. For example, if the cell is a liver cell, one could advantageously use the albumin gene control region (Pinkert et al., 1987, Genes and Devel. 1: 268-76); the alpha-feto-protein gene control region (Krumlauf et al., 1985, Mol. Cell Biol. 5: 1639-48; Hammer et al., 1987, Science 235: 53-58); or the alpha 1-antitrypsin gene control region (Kelsey et al., 1987, Genes and Devel. 1: 161-71), all of which are active in the liver.

The vectors of the invention can also contain an enhancer sequence that increases transcription in higher eukaryotic cells of nucleic acid encoding FoxM1B protein. Enhancers are cis-acting elements of DNA, are usually about 10-300 bp in length, and act on promoters to increase transcription. Enhancers are relatively orientation- and position-independent. They have been found within introns as well as within several kilobases both 5' and 3' to the transcription unit. Several enhancer sequences available from mammalian genes are known (*e.g*., enhancers from globin, elastase, albumin, alpha-feto-protein, insulin, transthyretin, and HNF-6 genes). An enhancer from a virus also can be used to increase expression of a gene. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to a nucleic acid molecule, it is typically located at a site 5' from the promoter.

Expression vectors of the invention may be constructed from a convenient starting vector such as a commercially available vector. Such vectors may or may not contain all of the desired flanking sequences. Where one or more of the flanking sequences described herein are not already present in the vector, they may be individually obtained and ligated into the vector. Methods used for obtaining each of the flanking sequences are well known to one skilled in the art.

After the vector has been constructed and a nucleic acid molecule encoding FoxM1B siRNA has been inserted into the proper site of the vector, the completed vector may be inserted into a suitable host cell or a target cell. The introduction of an expression vector encoding FoxM1B siRNA into a selected host cell or target cell may be accomplished by well-known methods including methods such as transfection, infection, calcium chloride, electroporation, microinjection, lipofection, DEAE-dextran method, or other known techniques as described above. The method selected will in part be a function of the type of host cell or target cell to be used. These methods and other suitable methods are well known to the skilled artisan, and are set forth, for example, in Sambrook et al., 2001, MOLECULAR CLONING: A LABORATORY MANUAL, 3d ed., Cold Spring Harbor Laboratory Press, Cold Spring Harbor, N.Y.

The term "host cell" is used to refer to a cell into which has been introduced, or that is capable of having introduced, a nucleic acid sequence and then of expressing a gene of interest. The term includes the progeny of the parent cell, whether or not the progeny is identical in morphology or in genetic make-up to the original parent, so long as the gene is present. In preferred embodiments, the host cell is a eukaryotic cell, more preferably a mammalian cell and most preferably a rodent or human cell.

Selection of an appropriate target cell will also depend on the various factors discussed above for selection of an appropriate host cell. In addition, a target cell can be selected based on the disease or condition that affects a patient who is to be treated by methods described herein.

The term "transfection" is used to refer to the uptake of foreign or exogenous DNA by a cell, and a cell has been "transfected" when the exogenous DNA has been introduced inside the cell. A number of transfection techniques are well known in the art and are disclosed herein. *See, e.g.,* Graham et al., 1973, Virology 52: 456; Sambrook et al., 2001, MOLECULAR CLONING: A LABORATORY MANUAL, 3d ed., Cold Spring Harbor Laboratory Press, Cold Spring Harbor, N.Y.; Davis et al., 1986, BASIC METHODS IN MOLECULAR BIOLOGY (Elsevier); and Chu et al., 1981, Gene 13: 197. Such techniques can be used to introduce an exogenous DNA into suitable host cells.

FoxM1B inhibitors as provided by the invention are species of short interfering RNA (siRNA). The term "short interfering RNA" or "siRNA" as used herein refers to a double stranded nucleic acid molecule capable of RNA interference or "RNAi", as disclosed, *for example,* in Bass, 2001, Nature 411: 428-429; Elbashir et al., 2001, Nature 411: 494-498; and Kreutzer et al., International PCT Publication No. WO 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No. WO 01/29058; Deschamps-Depaillette, International PCT Publication No. WO 99/07409; and Li et al., International PCT Publication No. WO 00/44914. As used herein, siRNA molecules need not be limited to those molecules containing only RNA, but may further encompass chemically modified nucleotides and non-nucleotides having RNAi capacity or activity.

RNA interference refers to the process of sequence-specific post-transcriptional gene silencing in animals mediated by short interfering RNAs (siRNA) (Fire et al., 1998, Nature 391:806). The presence of long dsRNAs in cells stimulates the activity of a ribonuclease III enzyme referred to as *"dicer." Dicer* is involved in processing of the long dsRNA into siRNA, which are short pieces of dsRNA (Berstein et al., 2001, Nature 409:363). Short interfering RNAs derived from *dicer* activity are typically about 21-23 nucleotides in length and comprise about 19 base pair duplexes. Dicer has also been implicated in the excision of 21 and 22 nucleotide small temporal RNAs (stRNA) from precursor RNA of conserved structure that are implicated in translational control (Hutvagner et al., 2001, Science 293:834). The RNAi response also features an endonuclease complex containing an siRNA, commonly referred to as an RNA-induced silencing complex (RISC), which mediates cleavage of single-stranded RNA having sequence homologous to the siRNA. Cleavage of the target RNA takes place in the middle of the region complementary to the guide sequence of the siRNA duplex (Elbashir et al., 2001, Genes Dev. 15:188).

Short interfering RNA mediated RNAi has been studied in a variety of systems. Fire *et al.* were the first to observe RNAi in *C*. *elegans* (1998, Nature 391:806). Wianny and Goetz described RNAi mediated by dsRNA in mouse embryos (1999, Nature Cell Biol. 2:70). Hammond *et al.* described RNAi in *Drosophila* cells transfected with dsRNA (2000, Nature 404:293). Elbashir *et al.* described RNAi induced by introduction of duplexes of synthetic 21-nucleotide RNAs in cultured mammalian cells including human embryonic kidney and HeLa cells (2001, Nature 411:494).

Recent work in *Drosophila* embryo lysates has revealed certain requirements for siRNA length, structure, chemical composition, and sequence that are essential to mediate efficient RNAi activity. These studies have shown that siRNA duplexes comprising 21 nucleotides are most active when containing two nucleotide 3'-overhangs. Furthermore, substitution of one or both siRNA strands with 2'-deoxy or 2'-O-methyl nucleotides abolishes RNAi activity, whereas substitution of 3'-terminal siRNA nucleotides with deoxy nucleotides was shown to be tolerated. Mismatch sequences in the center of the siRNA duplex were also shown to abolish RNAi activity. In addition, these studies also indicate that the position of the cleavage site in the target RNA is defined by the 5'-end of the siRNA guide sequence rather than the 3'-end (Elbashir et al., 2001, EMBO J. 20:6877). Other studies have indicated that a 5'-phosphate on the target-complementary strand of a siRNA duplex is required for siRNA activity and that ATP is utilized in cells to maintain the 5'-phosphate moiety on the siRNA (Nykanen et al., 2001, Cell 107:309). However siRNA molecules lacking a 5'-phosphate are active when introduced exogenously, suggesting that 5'-phosphorylation of siRNA constructs can *occur in vivo.*

A FoxM1B siRNA molecule described herein can be a double-stranded polynucleotide molecule comprising self-complementary sense and antisense regions, wherein the antisense region comprises a nucleotide sequence that is complementary to the nucleotide sequence of FoxM1B or a portion thereof, and the sense region has a nucleotide sequence corresponding to the FoxM1b nucleic acid sequence or a portion thereof, The FoxM1b siRNA molecule can be assembled from two separate oligonucleotides, where one strand is the sense strand and the other is the antisense strand, wherein the antisense and sense strands are self-complementary. The FoxM1B siRNA molecule can also be assembled from a single oligonucleotide having self-complementary sense and antisense regions linked by means of a nucleic acid based or non-nucleic acid-based linker. The FoxM1B siRNA molecule can be a polynucleotide can form a substantially symmetrical duplex, asymmetric duplex, hairpin, or asymmetric hairpin secondary structure, The FoxM1B siRNA molecule can also comprise a single stranded polynucleotide having nucleotide sequence complementary to the FoxM1B nucleotide sequence or a portion thereof, wherein the single stranded polynucleotide can further comprise a terminal phosphate group, such as a 5',3'-diphosphate or a 5'-phosphate as discussed, for example, in Martinez et al., 2002, Cell 110:563-574 and Schwarz et al., 2002, Molecular Cell 10:537-568.

A FoxM1b siRNA molecule described herein comprising a single stranded hairpin structure is preferably about 36 to about 70 nucleotides in length, having two complementary sequences of about 15 to about 30 nucleotides separated by a spacer sequence that allows hybridization of the complementary sequences. Thus, the single stranded hairpin structure has about 15 to about 30 base pairs comprising the duplex portion of the molecule. In one embodiment, the hairpin siRNA has about 18, 19, 20, or 21 base pairs in the duplex portion and a loop portion of a length that accommodates hybridization of the complementary siRNA sequences.

In preferred embodiments, a FoxM1B (or FoxM1) siRNA is designed and constructed as described in Example 1 herein, which describes production of a 19-basepair siRNA that corresponds to nucleotide residues 377-395 (SEQ ID NO: 1), 1066-1084 (SEQ ID NO: 2), 564-582 (SEQ ID NO: 3), or 223-261 (SEQ ID NO: 4) of the mouse FoxM1B coding sequence. A FoxM1B (or FoxM1) siRNA can also be produced using homologous sequences from human FoxM1B (or FoxM1) coding sequence. The FoxM1B siRNA described in Example 1 are exemplary FoxM1B siRNA molecules that have a nucleotide sequence as shown in SEQ ID NO: 1, 2, 3, or 4, Alternatively, FoxM1B siRNA can be constructed using the methods described in Elbashir et al. (2001, Genes Dev. 15:188-200; 2001, Nature 411:494-498).

In certain embodiments, the invention provides expression vectors comprising a nucleic acid sequence encoding at least one FoxM1B siRNA molecule of the invention, in a manner that allows expression of the FoxM1B siRNA molecule. For example, the vector can contain sequence(s) encoding both strands of a FoxM1B siRNA molecule comprising a duplex. The vector can also contain sequence(s) encoding a single nucleic acid molecule that is self-complomentary and thus forms a FoxM1B hairpin siRNA molecule. Non-limiting examples of such expression vectors are described in Paul et al., 2002, Nature Biotechnology 19:505; Miyagishi and Taira, 2002, Nature Biotechnology 19;497; Lee et al., 2002, Nature Biotechnology 19;500; and Novina et al., 2002, Nature Medicine, online publication June 3.

In other embodiments, the invention describes mammalian cells, for example, human cells, comprising an expression vector of the invention. In further embodiments, the expression vector comprised in said cells comprises a sequence for an siRNA molecule complimentary to at least a portion of human or mouse FoxMlB (or FoxM1) coding sequence, wherein expression of said siRNA in the cell inhibits FoxMlB expression therein. In other embodiments, expression vectors comprise a nucleic acid sequence encoding two or more siRNA molecules, which can be the same or different. In other embodiments, siRNA molecules, preferably FoxM1B-specific siRNAs molecules, are expressed from transcription units inserted into DNA or RNA vectors.

In certain embodiments, siRNA molecules described herein can comprise a delivery vehicle, including *inter alia* liposomes, for administration to a subject; carriers and diluents and their salts; and can be present in pharmaceutical compositions. Methods for the delivery of nucleic acid molecules are described, for example, in Akhtar et al., 1992, Trends Cell Bio. 2:139; Delivery Strategies for Antisense Oligonucleotide Therapeutics, ed. Akhtar, 1995, Maurer et al., 1999, Mol. Membr. Biol. 16:129-140; Hofland and Huang, 1999, Handb. Exp. Pharmacol., 137:165-192; and Lee et al., 2000, ACS Symp. Ser. 752;184-192.

Beigelman et al., U.S. Patent No. 6,395,713 and Sullivan et al., PCT WO 94/02595, further describe general methods for delivery of nucleic acid molecules into cells and tissues. These protocols can be utilized for the delivery of virtually any nucleic acid molecule into a cell. Nucleic acid molecules can be administered to cells by a variety of methods known to those of skill in the art, including, but not restricted to, encapsulation in liposomes, by iontophoresis, or by incorporation into other delivery vehicles, such as hydrogels, cyclodextrins, biodegradable nanocapsules, and bioadhesive microspheres, or by proteinaceous vectors (*see, for example,* O'Hare and Normand, International PCT Publication No. WO 00/53722).

Alternatively, the nucleic acid/vehicle combination can be locally delivered by direct injection or by use of an infusion pump. Direct injection of the nucleic acid molecules, whether subcutaneous, intramuscular, or intradermal, can take place using standard needle and syringe methodologies, or by needle-free technologies such as those described in Conry et al., 1999, Clin. Cancer Res. 5:2330-2337 and Barry et al., International PCT Publication No. WO 99/31262. Many examples in the art describe delivery methods of oligonucleotides by osmotic pump, *(see* Chun et al., 1998, Neuroscience Letters 257:135-138, D'Aldin et al., 1998, Mol. Brain Research 55:151-164, Dryden et al., 1998, J. Endocrinol. 157:169-175, Ghirnikar et al., 1998, Neuroscience Letters 247:21-24) or direct infusion (Broaddus et al., 1997, Neurosurg. Focus 3 article 4). Other delivery routes include, but are not limited to oral delivery (such as in tablet or pill form) and/or intrathecal delivery (Gold, 1997, Neuroscience 76:1153-1158). More detailed descriptions of nucleic acid delivery and administration are provided in Sullivan et al., PCT WO 94/02595, Draper et al., PCT WO93/23569, Beigelman et al., PCT WO99/05094, and Klimuk et al., PCT WO99/04819.

Alternatively, certain siRNA molecules of the invention can be expressed within cells from eukaryotic promoters *(see for example,* Izant and Weintraub, 1985, Science 229:345; McGarry and Lindquist, 1986, Proc. Natl. Acad. Sci USA 83:399; Scanlon et al., 1991, Proc. Natl. Acad. Sci. USA 88:10591-5; Kashani-Sabet et al., 1992, Antisense Res. Dev. 2:3-15; Dropulic et al., 1992, J. Virol. 66:1432-41; Weerasinghe et al., 1991, J. Virol. 65:5531-4; Ojwang et al., 1992, Proc. Natl. Acad. Sci, USA 89:10802-6; Chen et al., 1992, Nucleic Acids Res. 21:4581-9; Sarver et al., 1990, Science 247:1222-1225; Thompson et al., 1995, Nucleic Acids Res. 23:2259; Good et al., 1997, Gene Therapy 4: 45. Those skilled in the art will recognize that any nucleic acid can be expressed in eukaryotic cells using the appropriate DNA/RNA vector. The activity of such nucleic acids can be augmented by their release from the primary transcript by an enzymatic nucleic acid (Draper et al., PCT WO 93/23569, and Sullivan et al., PCT WO 94/02595; Ohkawa et al., 1992, Nucleic Acids Symp. Ser. 27:15-6; Taira et al., 1991, Nucleic Acids Res. 19:5125-30; Ventum et al., 1993, Nucleic Acids Res. 21:3249-55; Chowrira et al., 1994, J. Biol. Chem. 269:25856).

In another aspect of the invention, RNA molecules can be expressed from transcription units *(see for example,* Couture et al., 1996, TIG 12:510) inserted into DNA or RNA vectors. The recombinant vectors can be DNA plasmids or viral vectors. siRNA expressing viral vectors can be constructed based on, but not limited to, adeno-associated virus, retrovirus, adenovirus, or alphavirus. In another embodiment, pol III based constructs are used to express nucleic acid molecules of the invention *(see for example,* Thompson, U.S. Patent Nos. 5,902,880 and 6,146,886). The recombinant vectors capable of expressing the siRNA molecules can be delivered as described above, and persist in target cells. Alternatively, viral vectors can be used that provide for transient expression of nucleic acid molecules. Such vectors can be repeatedly administered as necessary. Once expressed, the siRNA molecule interacts with the target mRNA and generates an RNAi response. Delivery of siRNA molecule expressing vectors can be systemic, such as by intravenous or intramuscular administration, by administration to target cells ex-planted from a subject followed by reintroduction into the subject, or by any other means that would allow for introduction into the desired target cell (for a review, see Couture et al., 1996, TIG. 12:510)*.*

In one embodiment, the invention provides an expression vector comprising a nucleic acid sequence encoding at least one siRNA molecule of the invention. The expression vector can encode one or both strands of a siRNA duplex, or a single self-complementary strand that self hybridizes into an siRNA duplex. The nucleic acid sequences encoding the siRNA molecules can be operably linked in a manner that allows expression in a cell of the siRNA molecule (see *for example,* Paul et al., 2002, Nature Biotechnology 19:505; Miyagishi and Taira, 2002, Nature Biotechnology 19:497; Lee et al., 2002, Nature Biotechnology 19:500; and Novina et al., 2002, Nature Medicine, online publication June 3).

In another aspect, the invention describes an expression vector comprising: a) a transcription initiation region (e.g., eukaryotic pol I, II or III initiation region); b) a transcription termination region (e.g., eukaryotic pol I, II or III termination region); and c) a nucleic acid sequence encoding at least one of the siRNA molecules of the invention; wherein said sequence is operably linked to said initiation region and said termination region, in a manner that allows expression and/or delivery of the siRNA molecule. The vector can optionally include an open reading frame (ORF) for a protein operably linked on the 5' side or the 3'-side of the sequence encoding the siRNA of the invention; and/or an intron (intervening sequences).

Transcription or the siRNA molecule sequences can be driven from a promoter for eukaryotic RNA polymerase I (pol I), RNA polymerase II (pol II), or RNA polymerase III (pol III). Transcripts from pol II or pol III promoters are expressed at high levels in all cells; the levels of a given pol **II** promoter in a given cell type depends on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990, Proc. Natl. Acad Sci. USA 87:6743-7; Gao and Huang 1993, Nucleic Acids Res. 21:2867-72; Lieder et al., 1993, Methods Enzymol. 217:47-66; Zhou et al., 1990, Mol. Cell. Biol. 10:4529-37). Several investigators have demonstrated that nucleic acid molecules expressed from such promoters can function in mammalian cells (*e.g*. Kashani-Sabet et al., 1992, Antisense Res. Dev. 2:3-15; Ojwang et al., 1992, Proc. Natl. Acad. Sci. USA 89:10802-6; Chen et al., 1992, Nucleic Acids Res. 20:4581-9*;* Yu et al., 1993, Proc. Natl. Acad. Sci. USA 90:6340-4; L'Huillier et al., 1992, EMBO J. 11;411-8; Lisziewicz et al., 1993, Proc. Natl. Acad. Sci. U.S.A 90:8000-4; Thompson et al., 1995, Nucleic Acids Res. 23:2259; Sullenger and Cech, 1993, Science 262:1566). More specifically, transcription units such as the ones derived from genes encoding U6 small nuclear (snRNA), transfer RNA (tRNA) and adenovirus VA RNA are useful in generating high concentrations of desired RNA molecules such as siRNA in cells (Thompson et al., 1995, Nucleic Acids Res. 23:2259; Couture et al., 1996, TIG 12:510; Noonberg et al., 1994, Nucleic Acid Res. 22:2830; Noonberg et al., U.S. Patent No. 5,624,803; Good et al., 1997, Gene Ther. 4:45; Beigelman et al., International PCT Publication No. WO 96/18736. The above siRNA transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors) (for a review see Couture et al., 1996, TIG 12:510).

Expression vectors that are useful in the practice of the invention include expression vectors that comprise a nucleic acid sequence encoding two complementary sequences of an siRNA molecule separated by a small nucleotide spacer sequence, in a manner that allows expression of that siRNA molecule containing a hairpin loop. Generally, a useful expression vector comprises: a) a transcription initiation region; b) a transcription termination region; and c) a nucleic acid sequence encoding two complementary sequences of an siRNA molecule separated by a small nucleotide spacer sequence; wherein the sequence is operably linked to the initiation region and the termination region, in a manner that allows expression and/or delivery of the siRNA molecule containing the small hairpin loop.

Further the invention describes a method of inhibiting tumor growth in an animal comprising administering to the animal, which has at least one tumor cell present in its body, a therapeutically effective amount of a FoxM1B siRNA molecule, for example, as provided herein as SEQ ID NO: 1, 2, 3, and 4, for a therapautically effective period of time, wherein the FoxM1B siRNA molecule can inhibit FoxM1B gene expression.

In certain embodiments, the invention provides pharmaceutical compositions comprising a therapeutically effective amount of a FoxM1B siRNA molecule as provided herein as SEQ ID NO: 1, 2, 3, and 4, that inhibits FoxM1B expression in mammalian cells together with a pharmaceutically acceptable diluent, carrier, solubilizer, emulsifier, preservative and/or adjuvant. The invention further provides pharmaceutical compositions comprising a FoxM1B siRNA molecule as provided herein as SEQ ID NO: 1, 2, 3, and 4.

The term "agent" is used herein to denote a chemical compound, a mixture of chemical compounds, a biological macromolecule, or an extract made from biological materials.

The term "pharmaceutical composition" as used herein refers to a composition comprising a pharmaceutically acceptable carrier, excipient, or diluent and a chemical compound, peptide, or composition as described herein that is capable of inducing a desired therapeutic effect when properly administered to a patient.

The term "therapeutically effective amount" refers to the amount of growth hormone or a pharmaceutical composition or a compound identified in a screening method determined to produce a therapeutic response in a mammal. Such therapeutically effective amounts are readily ascertained by one of ordinary skill in the art and using methods as described herein.

As used herein, "substantially pure" means an object species that is the predominant species present (*i.e*., on a molar basis it is more abundant than any other individual species in the composition). In certain embodiments, a substantially purified fraction is a composition wherein the object species comprises at least about 50 percent (on a molar basis or on a weight or number basis) of all macromolecular species present. In certain embodiments, a substantially pure composition will comprise more than about 80%, 85%, 90%, 95%, or 99% of all macromolar species present in the composition. In certain embodiments, the object species is purified to essential homogeneity (wherein contaminating species cannot be detected in the composition by conventional detection methods) wherein the composition consists essentially of a single macromolecular species.

The term "patient" includes human and animal subjects.

As used herein, the terms "tumor growth" and "tumor cell proliferation" are used to refer to the growth of tumor cells. The term "tumor cell" as used herein refers to a cell that is neoplastic. A tumor cell can be benign, *i.e*. one that does not form metastases and does not invade and destroy adjacent normal tissue, or malignant, *i.e*. one that invades surrounding tissues, is capable of producing metastases, may recur after attempted removal, and is likely to cause death of the host. Preferably a tumor cell that is subjected to a use of the invention is an epithelial-derived tumor cell, such as a tumor cell derived from skin cells, lung cells, intestinal epithelial cells, colon epithelial cells, testes cells, breast cells, prostate cells, brain cells, bone marrow cells, blood lymphocytes, ovary cells or thymus cells.

Acceptable formulation materials preferably are nontoxic to recipients at the dosages and concentrations employed. The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides, disaccharides, and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20 and polysorbate 80, Triton, trimethamine, lecithin, cholesterol, or tyloxapal); stability enhancing agents (such as sucrose or sorbitol); tonicity enhancing agents (such as alkali metal halides, preferably sodium or potassium chloride, mannitol, or sorbitol); delivery vehicles; diluents; excipients and/or pharmaceutical adjuvants. *See, for example,* REMINGTON's PHARMACEUTICAL SCIENCES, 18th Edition, (A.R. Gennaro, ed.), 1990, Mack Publishing Company.

Optimal pharmaceutical compositions can be determined by one skilled in the art depending upon, for example, the intended route of administration, delivery format and desired dosage. *See, for* example. REMINGTON's PHARMACEUTICAL SCIENCES, *Id.* Such compositions may influence the physical state, stability, rate of *in vivo* release and rate of *in vivo* clearance of antibodies.

Primary vehicles or carriers in a pharmaceutical composition can include, but are not limited to, water for injection, physiological saline solution or artificial cerebrospinal fluid, possibly supplemented with other materials common in compositions for parenteral administration. Neutral buffered saline or saline mixed with serum albumin are further exemplary vehicles. Pharmaceutical compositions can comprise Tris buffer of about pH 7.0-8.5, or acetate buffer of about pH 4.0-5.5, which may further include sorbitol or a suitable substitute therefor. Pharmaceutical compositions of the invention may be prepared for storage by mixing the selected composition having the desired degree of purity with optional formulation agents (REMINGTON'S PHARMACEUTICAL SCIENCES, Id) in the form of a lyophilized cake or an aqueous solution. Further, the FoxM1B-inhibiting siRNAs may be formulated as a lyophilizate using appropriate excipients such as sucrose.

Formulation components are present in concentrations that axe acceptable to the site of administration. Buffers are advantageously used to maintain the composition at physiological pH or at a slightly lower pH, typically within a pH range of from about 5 to about 8.

The pharmaceutical compositions of the invention can be delivered parenterally. When parenteral administration is contemplated the therapeutic compositions for use in this invention may be in the form of a pyrogen-free, parenterally acceptable aqueous solution comprising the desired siRNA of the invention. Preparation can involve the formulation of the desired molecule with an agent, such as injectable microspheres, bio-erodible particles, polymeric compounds (such as polylactic acid or polyglycolic acid), beads or liposomes, that may provide controlled or sustained release of the product which may then be delivered via a depot injection. Formulation with hyaluronic acid has the effect of promoting sustained duration in the circulation. Implantable drug delivery devices may be used to introduce the desired molecule.

The compositions may be formulated for inhalation. In these embodiments, a compound identified in a screening method of the invention or a FoxM1B siRNA disclosed herein is formulated as a dry powder for inhalation, or inhalation solutions may also be formulated with a propellant for aerosol delivery, such as by nebulization. Pulmonary administration is further described is PCT Application No. PCT/US94/001875, which describes pulmonary delivery of chemically modified proteins.

The pharmaceutical compositions of the invention can be delivered through the digestive tract, such as orally. The preparation of such pharmaceutically acceptable compositions is within the skill of the art. A FoxM1B siRNA disclosed herein that are administered in this fashion may be formulated with or without those carriers customarily used in the compounding of solid dosage forms such as tablets and capsules. A capsule may be designed to release the active portion of the formulation at the point in the gastrointestinal tract when bioavailability is maximized and pre-systemic degradation is minimized. Additional agents can be included to facilitate absorption of the FoxM1B siRNA disclosed herein. Diluents, flavorings, low melting point waxes, vegetable oils, lubricants, suspending agents, tablet disintegrating agents, and binders may also be employed.

A pharmaceutical composition may involve an effective quantity of a FoxM1B siRNA disclosed herein in a mixture with non-toxic excipients that are suitable for the manufacture of tablets. By dissolving the tablets in sterile water, or another appropriate vehicle, solutions may be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical compositions are evident to those skilled in the art, including formulations involving a FoxM1B inhibitor disclosed herein or compounds of the invention in sustained- or controlled-delivery formulations. Techniques for formulating a variety of other sustained- or controlled-delivery means, such as liposome carriers, bio-erodible microparticles or porous beads and depot injections, are also known to those skilled in the art. *See, for example,* PCT Application No. PCT/US93/00829, which describes the controlled release of porous polymeric microparticles for the delivery of Pharmaceutical compositions. Sustained-release preparations may include semipermeable polymer matrices in the form of shaped articles, *e.g*. films, or microcapsules, polyesters, hydrogels, polylactides (U.S. 3,773,919 and EP 058,481), copolymers of L-glutamic acid and gamma ethyl-L-glutamate (Sidman et al., 1983, Biopolymers 22: 547-556), poly (2-hydroxyethyl-methacrylate) (Langer et al., 1981, J. Biomed. Mater. Res. 15: 167-277) and Langer, 1982, Chem. Tech. 12: 98-105), ethylene vinyl acetate (Langer et al., id.) or poly-D(-)-3-hydroxybutyric acid (EP 133,988). Sustained release compositions may also include liposomes, which can be prepared by any of several methods known in the *art. See e.g.,* Eppstein et al., 1985, Proc. Natl. Acad. Sci. USA 82: 3688-3692; EP 036,676; EP 088,046 and EP 143,949.

The pharmaceutical composition to be used for *in vivo* administration typically is sterile. In certain embodiments, this may be accomplished by filtration through sterile filtration membranes. In certain embodiments, where the composition is lyophilized, sterilization using this method may be conducted either prior to or following lyophilization and reconstitution. In certain embodiments, the composition for parenteral administration may be stored in lyophilized form or in a solution. In certain embodiments, parenteral compositions generally are placed into a container having a sterile access port, for example, an intravenous solution bag or vial having a stopper pierceable by a hypodermic injection needle.

Once the pharmaceutical composition of the invention has been formulated, it may be stored in sterile vials as a solution, suspension, gel, emulsion, solid, or as a dehydrated or lyophilized powder. Such formulations may be stored either in a ready-to-use form or in a form (*e.g*., lyophilized) that is reconstituted prior to administration.

The present invention describes kits for producing a single-dose administration unit. Said kits may each contain both a first container having a dried protein compound identified in a screening method of the invention and a second container having an aqueous formulation, including for example single and multi-chambered pre-filled syringes (*e*.*g*., liquid syringes, lyosyringes or needle-free syringes).

The effective amount of a pharmaceutical composition of the invention to be employed therapeutically will depend, for example, upon the therapeutic context and objectives. One skilled in the art will appreciate that the appropriate dosage levels for treatment, according to certain embodiments, will thus vary depending, in part, upon the molecule delivered, the indication for which the pharmaceutical composition is being used, the route of administration, and the size (body weight, body surface or organ size) and/or condition (the age and general health) of the patient. A clinician may titer the dosage and modify the route of administration to obtain the optimal therapeutic effect.

The dosing frequency will depend upon the pharmacokinetic parameters of a FoxM1B siRNA disclosed herein. For example, a clinician administers the siRNA until a dosage is reached that achieves the desired effect. The composition may therefore be administered as a single dose, or as two or more doses (which may or may not contain the same amount of the desired molecule) over time, or as a continuous infusion via an implantation device or catheter. Further refinement of the appropriate dosage is routinely made by those of ordinary skill in the art and is within the ambit of tasks routinely performed by them. Appropriate dosages may be ascertained through use of appropriate dose-response data.

Administration routes for the pharmaceutical compositions of the invention include orally, through injection by intravenous, intraperitoneal, intracerebral (intraparenchymal), intracerebroventricular, intramuscular, intra-ocular, intraarterial, intraportal, or intralesional routes; by sustained release systems or by implantation devices. The pharmaceutical compositions may be administered by bolus injection or continuously by infusion, or by implantation device. The pharmaceutical composition also can be administered locally via implantation of a membrane, sponge or another appropriate material onto which the desired molecule has been absorbed or encapsulated. Where an implantation device is used, the device may be implanted into any suitable tissue or organ, and delivery of the desired molecule may be via diffusion, timed-release bolus, or continuous administration.

In certain embodiments, it may be desirable to use a FoxM1B siRNA disclosed herein or pharmaceutical compositions comprising a FoxM1B siRNA of the invention in an *ex vivo* manner. In such instances, cells, tissues or organs that have been removed from the patient are exposed to pharmaceutical compositions of the invention or an siRNA disclosed herein after which the cells, tissues and/or organs are subsequently implanted back into the patient.

Pharmaceutical compositions of the invention can be administered alone or in combination with other therapeutic agents, in particular, in combination with other cancer therapy agents. Such agents generally include radiation therapy or chemotherapy. Chemotherapy, for example, can involve treatment with one or more of the following agents: anthracyclines, taxol, tamoxifene, doxorubicin, 5-fluorouracil, and other drugs known to one skilled in the art.

Introducing an siRNA of the invention into cells can be accomplished using any method known in the art or as described herein. For example, local delivery of a FoxM1B siRNA can be accomplished by direct injection or by other appropriate viral or non-viral delivery vectors. (Hefti, 1994, Neurobiology 25:1418-35.) For example, a nucleic acid molecule encoding a FoxM1B polypeptide may be contained in an adeno-associated virus (AAV) vector for delivery to the targeted cells *(see, e.g.,* Johnson, PCT Pub. No. WO 95/34670; PCT App. No. PCT/US95/07178). The recombinant AAV genome used according to the teachings of the invention typically contains AAV inverted terminal repeats flanking a DNA sequence encoding a FoxM1B siRNA operatively linked to functional promoter and polyadenylation sequences.

Alternative suitable viral vectors include, but are not limited to, retrovirus, adenovirus, herpes simplex virus, lentivirus, hepatitis virus, parvovirus, papovavirus, poxvirus, alphavirus, coronavirus, rhabdovirus, paramyxovirus, and papilloma virus vectors. U.S. Patent No. 5,672,344 describes an *in vivo* viral-mediated gene transfer system involving a recombinant neurotrophic HSV-1 vector. U.S. Patent No. 5,399,346 provides examples of a process for providing a patient with a therapeutic protein by the delivery of human cells that have been treated *in vitro* to insert a DNA segment encoding a therapeutic protein. Additional methods and materials for the practice of gene therapy techniques are described in U.S. Patent Nos. 5,631,236 (involving adenoviral vectors), 5,672,510 (involving retroviral vectors), and 5,635,399 (involving retroviral vectors expressing cytokines).

Nonviral delivery methods include, but are not limited to, liposome-mediated transfer, naked DNA delivery (*e.g*., by direct injection), receptor-mediated transfer (ligand-DNA complex), electroporation, calcium phosphate precipitation, and microparticle bombardment (*e.g*., gene gun). Gene therapy materials and methods may also include inducible promoters, tissue-specific enhancer-promoters, DNA sequences designed for site-specific integration, DNA sequences capable of providing a selective advantage over the parent cell, labels to identify transformed cells, negative selection systems and expression control systems (safety measures), cell-specific binding agents (for cell targeting), cell-specific internalization factors, and transcription factors to enhance expression by a vector as well as methods of vector manufacture. Such additional methods and materials for the practice of gene therapy techniques are described in U.S. Patent Nos. 4,970,154 (involving electroporation techniques), 5,679,559 (describing a lipoprotein-containing system for gene delivery), 5,676,954 (involving liposome carriers), 5,593,875 (describing methods for calcium phosphate transaction), and 4,945,050 (describing a process wherein biologically active particles are propelled at cells at a speed whereby the particles penetrate the surface of the cells and become incorporated into the interior of the cells), and PCT Pub. No. WO 96/40958 (involving nuclear ligands).

### EXAMPLES

### Example 1

### FoxM1B siRNA reduces both FoxM1B transcriptional activity

Recent mouse genetic studies demonstrated that Alb-Cre *Foxm1b* -/- hepatocytes fail to proliferate and are highly resistant to developing Hepatocellular Carcinoma (HCC) following DEN/PB liver tumor induction (Kalinichenko et al. 2004 Genes & Dev. 18:830-850). In order to develop a method to inhibit expression of FoxM1b protein, an algorithm was used (Dharmacon Research, Lafayette, Colorado) to synthesize four 19-nucleotide short interfering RNA (siRNA) duplexes specific to the human FoxM1b cDNA that contained an additional symmetric 2-uracil (U) 3' overhang at each end. These four Foxm1b siRNA sequences and their nucleotide positions within the human Foxm1b cDNA sequence are shown in Table 1.

**Table 1. Targeting sequences and position of human Foxm1b targeting sequence.**

| Designation | FoxM1b Nucleotide position | Targeting sequence |
|---|---|---|
| siFoxM1b#1; SEQ ID NO: 1 | 377 to 395 | **5'-caa cag gag ucu aau caa g uu-3'** |
| siFoxM1b#2; SEQ ID NO: 2 | 1066 to 1084 | **5'-gga cca cuu ucc cua cuu u uu-3'** |
| siFoxM1b#3; SEQ ID NO: 3 | 564 to 582 | **5'-gua gug ggc cca aca aau u uu-3'** |
| siFoxM1b#4; SEQ ID NO: 4 | 223 to 261 | **5'-gcu ggg auc aag auu auu a uu-3'** |

| | | |
|---|---|---|
| Four different 19-nucleotide short interfering RNA (siRNA) duplexes, each specific to the Human FoxM1b cDNA (indicated is the nucleotide position in the Human FoxM1b cDNA), were synthesized. Each of the siRNA duplexes contained an additional symmetric 2-uracil (U) 3' overhang at each end to enhance stability. | | |

These FoxM1b siRNAs were designated siFoxM1b #1 to #4; also used was a FoxM1b siRNA pool containing a mixture of these four FoxM1b siRNAs (siFoxM1b pool). A TetO-GFP-FoxM1b clone C3 U2OS cell line, in which the addition of Doxycycline (Dox) to the tissue culture medium stimulated expression of the Green Fluorescent Protein (GFP)-Foxm1b fusion protein (Kalinichenko et al., 2004, Genes & Dev. 18:830-850) was used to analyze activity of the siFoxM1b molecules. A dose response curve for these FoxM1b siRNA duplexes was performed by transfecting increasing amounts of either the individual FoxM1b siRNAs (Table 1, siFoxM1b #1 to #4) or the siFoxM1b pool into the TetO-GFP-FoxM1b clone C3 U2OS cell line following Doxycycline induction of the GFP-FoxM1b protein. The siRNAs were transfected using Lipofectamine 2000 (Invitrogen Corp., Carlsbad, CA). Protein extracts were prepared 48 hours after transfection and then analyzed for GFP-FoxM1b protein levels by Western Blot analysis using a monoclonal antibody against GFP (Fig. 1A). These analyses demonstrated that transfection of 100 nM of each of the FoxM1b siRNAs reduced Dox induced expression of the GFP-FoxM1b protein in the TetO-GFP-FoxM1b clone C3 U2OS cell line (Fig. 1A).

The siFoxM1b #2 duplex was the most effective in decreasing FoxM1b protein expression because transfection of only 25 nM of the FoxM1b siRNA #2 was sufficient to completely inhibit expression of induced GFP-FoxM1b protein (Fig. 1A). Previous studies demonstrated that the Foxm1b protein was essential for transcription of the Cdc25B phosphatase gene, whose expression is required for Cdkl activation (Wang et al. 2002 Proc. Natl. Acad. Sci USA. 99:6881-16886). Consistent with these studies, induced levels of the GFP-FoxM1b protein stimulated expression of the Cdc25B protein compared to untreated clone C3 U20S cell line (Fig. 1B, compare - and + Dox). Furthermore, transfection of increasing amounts of either siFoxM1b #1 or siFoxM1b #2 into Dox treated clone C3 U2OS cell line demonstrated that siFoxM1b #2 effectively diminished expression of the Cdc25B protein (Fig. 1B), a finding consistent with the ability of this siRNA to diminish GFP-FoxM1b protein levels (Fig. 1A).

The Clone C3 U2OS cell line was transfected with equal amounts of these FoxM1b siRNAs, and protein extracts were isolated 48 hours after transfection and Western blot analysis was used to measure induced levels of GFP-Foxm1b protein and potential FoxM1b target proteins involved in orchestrating mitosis. One such target is Aurora B Kinase, which is involved in orchestrating mitosis and has been shown to have an 85% reduction in expression in *Foxm1b* deficient (-/-) embryonic liver (Krupezak-Hollis et al. 2004 Dev. Biol., In press). The Western blot analysis demonstrated that doxycycline induction of the GFP-FoxM1b protein caused increased expression of the Aurora B Kinase protein compared to untreated clone C3 U2OS cell extracts (Fig. 2, compare 0 siRNA -Dox and + Dox). These studies indicated that all of the Foxm1b siRNA diminished expression of induced GFP-FoxM1b protein levels, and siFoxM1b #2 completely inhibited expression levels of GFP-FoxM1b protein (Fig. 2). Transfection of FoxM1b siRNA #1, #2 or #3 effectively diminishes levels of Aurora B Kinase protein and only FoxM1b siRNA #1 and #2 effectively reduced expression of M-phase regulator Polo-like kinase (Plk-1) protein (Fig. 2). In contrast, siRNA mediated reduction in GFP-FoxM1b levels did not influence expression of Inner Centromere Protein (INCENP), which is a chromosome passenger protein involved in mitosis (Fig. 2). Interestingly, when expression of GFP-FoxM1b was inhibited by the FoxM1b siRNAs, expression of E2F1 protein was also reduced.

Transfection of the Foxm1b siRNA #1, #2 and #3 also caused a significant reduction in cell growth rate compared to untreated controls or the Foxm1b siRNA #4, the latter of which is a less efficient inhibitor of GFP-FoxM1b protein expression (Fig. 3). Transfection of U20S cells with the FoxM1b siRNA pool caused a significant reduction in cellular DNA replication with an increased number of cells accumulating in the G2/M-phase of the cell cycle as determined by flow cytometry (Fig. 4). These results suggested that siRNA mediated reduction in FoxM1b expression caused a G2/M block, indicating that FoxM1b is required for progression into mitosis. Furthermore, transfection of the Foxm1b siRNA #1, #2, #3 or siRNA pool into Dox-induced C3 clone U2OS cell line caused a significant reduction in anchorage-independent growth as evidenced by reduction in the number of cell colonies growing on soft agar (Fig. 5A through 5B). These studies indicated that the Foxm1b siRNA was an effective reagent to diminish Foxm1b mediated cellular proliferation and cellular transformation (growth on soft agar).

## Claims

1. siRNA molecule that inhibits FoxM1 B activity in a tumor cell, wherein the siRNA comprises a nucleotide sequence shown in SEQ ID NO. 1.2,3 or 4.

2. siRNA molecule of claim 1, wherein the siRNA comprises a single self-complementary RNA strand.

3. siRNA molecule of claim 1, wherein the siRNA comprises two separate complementary RNA strands.

4. Expression vector comprising a nucleotide sequence encoding the siRNA molecule of claim 1.

5. Expression vector of claim 4, wherein the expression vector is a viral vector.

6. Viral vector of claim 5, wherein the viral vector is an adeno-associated virus, retrovirus, adenovirus, or alphavirus.

7. Use of the siRNA molecule of claim 1 for the preparation of a medicament for treating a cancer patient.

8. Use of the siRNA molecule of claim 1 for the preparation of a medicament for inhibiting proliferation of a tumor cell, wherein the cell is contacted with the siRNA.

9. Pharmaceutical composition comprising the siRNA molecule according to claim 1.

10. Use of the pharmaceutical composition of claim 9 for the preparation of a medicament for treating a cancer patient.

11. Use of the pharmaceutical composition of claim 9 for the preparation of a medicament for inhibiting proliferation of a tumor cell, wherein the cell is contacted with the siRNA.

12. siRNA molecule of claim 1 for use in the treatment of cancer.

13. siRNA molecule of claim 1 for use in inhibiting proliferation of a tumor cell.

14. Pharmaceutical composition of claim 9 for use in treating cancer.

15. Pharmaceutical composition of claim 9 for use in inhibiting proliferation of a tumor cell.

16. Method of inhibiting proliferation of a tumor cell comprising the step of contacting the cell with the siRNA molecule of claim 1, wherein the method is not a method of treatment of the human or animal body by surgery or therapy.

## Patentansprüche

1. siRNA-Molekül, das die FoxM1 B-Aktivität in einer Tumorzelle hemmt, wobei die siRNA eine in der SEQ ID NO. 1, 2, 3 oder 4 gezeigte Nucleotidsequenz umfasst.

2. siRNA-Molekül nach Anspruch 1, wobei die siRNA einen einzelnen, selbstkomplementären RNA-Strang umfasst.

3. siRNA-Molekül nach Anspruch 1, wobei die siRNA zwei getrennte, komplementäre RNA-Stränge umfasst.

4. Expressionsvektor, umfassend eine Nucleotidsequenz, die das siRNA-Molekül nach Anspruch 1 kodiert.

5. Expressionsvektor nach Anspruch 4, wobei es sich bei dem Expressionsvektor um einen viralen Vektor handelt.

6. Viraler Vektor nach Anspruch 5, wobei es sich bei dem viralen Vektor um ein Adeno-assoziiertes Virus, Retrovirus, Adenovirus oder Alphavirus handelt.

7. Verwendung des siRNA-Moleküls nach Anspruch 1 zur Herstellung eines Medikaments zum Behandeln eines Krebspatienten.

8. Verwendung des siRNA-Moleküls nach Anspruch 1 zur Herstellung eines Medikaments zum Hemmen der Proliferation einer Tumorzelle, wobei die Zelle mit der siRNA in Kontakt gebracht wird.

9. Pharmazeutische Zusammensetzung, umfassend das siRNA-Molekül nach Anspruch 1.

10. Verwendung der pharmazeutischen Zusammensetzung nach Anspruch 9 zur Herstellung eines Medikaments zum Behandeln eines Krebspatienten.

11. Verwendung der pharmazeutischen Zusammensetzung nach Anspruch 9 zur Herstellung eines Medikaments zum Hemmen der Proliferation einer Tumorzelle, wobei die Zelle mit der siRNA in Kontakt gebracht wird.

12. siRNA-Molekül nach Anspruch 1 zur Verwendung bei der Behandlung von Krebs.

13. siRNA-Molekül nach Anspruch 1 zur Verwendung beim Hemmen der Proliferation einer Tumorzelle.

14. Pharmazeutische Zusammensetzung nach Anspruch 9 zur Verwendung beim Behandeln von Krebs.

15. Pharmazeutische Zusammensetzung nach Anspruch 9 zur Verwendung beim Hemmen der Proliferation einer Tumorzelle.

16. Verfahren zum Hemmen der Proliferation einer Tumorzelle, umfassend den Schritt, die Zelle mit dem siRNA-Molekül nach Anspruch 1 in Kontakt zu bringen, wobei es sich bei dem Verfahren nicht um ein Verfahren der Behandlung des menschlichen oder tierischen Körpers durch Chirurgie oder Therapie handelt.

## Revendications

1. Molécule d'ARNsi qui inhibe l'activité de FoxM1B dans une cellule tumorale, l'ARNsi comprenant une séquence de nucléotides présentée dans la SEQ ID NO : 1, 2, 3 ou 4.

2. Molécule d'ARNsi selon la revendication 1, l'ARNsi comprenant un seul brin d'ARN auto-complémentaire.

3. Molécule d'ARNsi selon la revendication 1, l'ARNsi comprenant deux brins séparés d'ARN complémentaires.

4. Vecteur d'expression comprenant une séquence de nucléotides codant la molécule d'ARNsi de la revendication 1%

5. Vecteur d'expression selon la revendication 4, le vecteur d'expression étant un vecteur viral.

6. Vecteur viral selon la revendication 5, le vecteur viral étant un virus adéno-associé, un rétrovirus, un adénovirus, ou un alphavirus.

7. Utilisation de la molécule d'ARNsi de la revendication 1 pour la préparation d'un médicament destiné à traiter un patient cancéreux.

8. Utilisation de la molécule d'ARNsi de la revendication 1 pour la préparation d'un médicament destiné à inhiber la prolifération d'une cellule tumorale, dans laquelle la cellule est mise en contact avec l'ARNsi.

9. Composition pharmaceutique comprenant la molécule d'ARNsi de la revendication 1.

10. Utilisation de la composition pharmaceutique de la revendication 9 pour la préparation d'un médicament destiné à traiter un patient cancéreux.

11. Utilisation de la composition pharmaceutique de la revendication 9 pour la préparation d'un médicament destiné à inhiber la prolifération d'une cellule tumorale, dans laquelle la cellule est mise en contact avec l'ARNsi.

12. Molécule d'ARNsi de la revendication 1, pour utilisation dans le traitement d'un cancer.

13. Molécule d'ARNsi de la revendication 1, pour utilisation dans l'inhibition de la prolifération d'une cellule tumorale.

14. Composition pharmaceutique de la revendication 9, pour utilisation dans le traitement d'un cancer.

15. Composition pharmaceutique de la revendication 9, pour utilisation dans l'inhibition de la prolifération d'une cellule tumorale.

16. Procédé pour inhiber la prolifération d'une cellule tumorale, comprenant l'étape consistant à mettre la cellule en contact avec la molécule d'ARNsi de la revendication 1, lequel procédé n'est pas une méthode de traitement du corps humain ou animal par chirurgie ou thérapie.
